# EUROPEAN PATENT SPECIFICATION

(11) **EP 0 626 161 B1**
(45) Date of publication and mention of the grant of the patent: **22.04.1998**
(21) Application number: 94108162.2
(22) Date of filing: 26.05.1994
(51) Int. Cl.: A61F 13/15

(54) **Absorbent article and method of manufacturing the same**
Absorbierender Artikel und dessen Herstellungsmethode
Article absorbant et méthode pour sa fabrication

(30) Priority: 26.05.1993 JP 145362/93
(43) Date of publication of application: 30.11.1994
(73) Proprietor: JAPAN ABSORBENT TECHNOLOGY INSTITUTE, Chuo-ku, Tokyo (JP)
(72) Inventor: Suzuki, Migaku, Kamakura-shi, Kanagawa (JP); Fukui, Hiroaki, Kawaguchi-shi, Saitama (JP)
(74) Representative: Klingseisen, Franz, Dipl.-Ing.

(56) References cited:
- EP-A- 0 437 771
- EP-A- 0 510 715
- US-A- 4 662 877

## Description

The invention relates to an absorbent article according to the preamble of claim 1.

Such an absorbent article is known from EP-A-0 437 771 or EP-A-0 405 575, wherein the article comprises a water-permeable topsheet, a water-permeable backsheet and a water-absorbent core therebetween. Elastic members are bonded to the surface of the backsheet.

EP-A-0 510 715 discloses the production of parts for an absorbent article, wherein elastic members which form a cross-over portion between leg holes are cut along the cross-over portion which is bonded to the sheet material. In this way separate articles are produced from a continuous sheet material.

Absorbent articles, particularly infant and adult diapers have recently gained increased acceptance from consumers for their advantageous characteristics of stability during use and reduced leakage.

In order for tapeless absorbent articles to fully exhibit their advantageous characteristics, they need to fit snugly to a user's body, particularly at leg hole portions thereof. To this end, such tapeless absorbent articles have elastically stretchable ruffles or leg gathers, as they are generally called, along peripheries of the leg holes.

One example of such tapeless absorbent articles with leg gathers is disclosed in Japanese Kokai Patent Hei 3-195558. Two sets of elastic members are attached along the peripheries of the leg holes to form leg gathers. Each set of elastic members extends along one leg hole from a front end of the leg hole to a midpoint of the leg hole, and continuously extends transversely of a central region (crotch region) of the article to a midpoint of the other leg hole to form a cross-over portion. From the midpoint of the other leg hole, each set of elastic members further extends therealong to a rear end of the other leg hole. Those two sets of elastic members are arranged so as to define a somewhat X-shaped configuration.

Such arrangement of the elastic members advantageously facilitates a continuous manufacturing process of the absorbent articles. Specifically, a liquid impermeable sheet in a continuous web form is continuously transported in one direction. Continuous elastic strands are fed onto the sheet in a sinusoidal configuration for securement thereto. The resultant combination is cut at a predetermined interval to form a composite backsheet incorporating the elastic members secured thereto.

The elastic members arranged along peripheries of the leg holes in the aforementioned, conventional tapeless absorbent article function to form leg gathers therealong. However, the elastic members extending transversely of the crotch region not only are non-functional waste material but cause the absorbent article to deform at the crotch region when they elastically contract.

An attempt to remedy such disadvantages has been made by adjusting a stretching or contracting rate of the elastic members to be lower at the crotch region than along the leg holes. However, it complicates a manufacturing process, and lowers productivity to intermittently change the stretching rate of the elastic members during the continuous manufacturing process wherein the continuous elastic members are fed along predetermined lines on a web for securement thereto. The elastic members tend to impair flexibility and comfort to a wearer during use at the crotch region of the absorbent article where it is desired to be sufficiently flexible to provide comfort to the wearer during use. Furthermore, added elastic material for the elastic members is required at the cross-over region when they are secured to the backsheet at the lower stretching rate. This results in an increased material use as those elastic members at the cross-over region are not necessary.

The two sets of elastic members secured to the backsheet create bundle-like bunches at the crotch region of the absorbent article, which results in the article having a poor appearance.

It is an object of the present invention to provide an improved absorbent article which is capable of eliminating the above-described disadvantages that conventional articles possess, so that any undesirable deformation of the crotch region due to the contracting force of the elastic members is avoided, and flexibility thereat is insured.

This object is achieved by the features in the characterizing part of claim 1.

In accordance with the present invention, there is provided an absorbent article which is provided with a main body having a waist hole and a pair of leg holes. The main body comprises a liquid impermeable backsheet, a liquid permeable topsheet and an absorbent core interposed between the backsheet and the topsheet. The absorbent article is further provided with a waist gather disposed along the waist hole, and a leg gather disposed along each of the pair of leg holes.

The topsheet may include two layers of sheet materials, and two sets of elastic members interposed between the two sheets of material to form a composite sheet. Each of the two sets of elastic members extend along a periphery of a first leg hole from a front end thereof to a midpoint thereof, and continuously extend transversely to a midpoint of a second leg hole to form a cross-over portion. From the midpoint, each set of elastic members extend to a rear end of the second leg hole, so that the two sets of elastic members are arranged to define an X-shaped configuration. The two sets of the elastic members are secured in a stretched state to the sheet materials along areas that extend along the peripheries of the leg holes. The cross-over portions thereof are not secured to the sheet materials, and are separated so that they snap back toward cross portions of the two sets of the elastic members to define tail portions extending from the cross portions.

As such, the absorbent article in accordance with the present invention has the elastic members extending around leg holes to form leg gathers. The elastic members do not cross the crotch region of the article. Accordingly, the absorbent article provides a snug fit and comfort to the wearer during use, with its leg gathers effectively preventing leakage from the article. The leg elastic members are not secured to the backsheet, so that the absorbent article provides a good appearance.

In one embodiment of the present invention, the topsheet has a through aperture in the crotch region of the absorbent article. The aperture serves as an inlet void of a pocket for communicating urine and fecal material into the pocket defined between the topsheet and the backsheet. This provides comfort to the wearer during use.

The present invention further provides a method for manufacturing a tapeless absorbent article which comprises a main body having a waist hole and a pair of leg holes and comprising a liquid permeable topsheet, a liquid impermeable backsheet and an absorbent core interposed between the topsheet and the backsheet, a waist gather disposed along the waist hole, and a leg gather disposed along each of the leg holes.

Two sets of elastic members in their stretched conditions are positioned, between two sheets of material in elongated web forms, in sinusoidal configurations with an inverted phase relation to each other, so that the two sets of elastic members define a number of elongated oblong areas therebetween. The elastic members are bonded to the sheet material in alternate ones of the oblong areas, and are cut or separated in the oblong areas in which they are not secured so that they snap back toward crossing portions thereof.

The topsheet is combined with the backsheet prior to bi-folding of the resultant combination along a transverse center line. The bi-folded resultant combination is severed along predetermined cutting lines.

The present method can advantageously provide a highly productive process which is capable of securing elastic members to sheet materials without varying the feed rate of the sheet materials.

One embodiment of the present invention will be explained hereinafter with reference to the drawings.

### BRIEF DESCRIPTION OF THE DRAWINGS

FIGURE 1 is a perspective view illustrating one embodiment of an absorbent article in accordance with the present invention;
FIGURE 2 is a plan view of the absorbent article of FIGURE 1 prior to bi-folding and securing the respective side panels thereof;
FIGURE 3 is an explanatory view illustrating an intermediate product during the process of manufacturing a topsheet for use in an absorbent article of the present invention;
FIGURE 4A is a fragmentary cross-sectional view taken along a line A-A of Figure 3;
FIGURE 4B is a cross-sectional view taken along a line B-B of Figure 3;
FIGURE 5 is an explanatory view illustrating an intermediate product with portions of elastic members separated.
FIGURE 6A, 6B, 6C, 6D respectively illustrate plan views of various embodiments of topsheets for use in an absorbent article in accordance with the present invention.

### DESCRIPTION OF THE PREFERRED EMBODIMENT

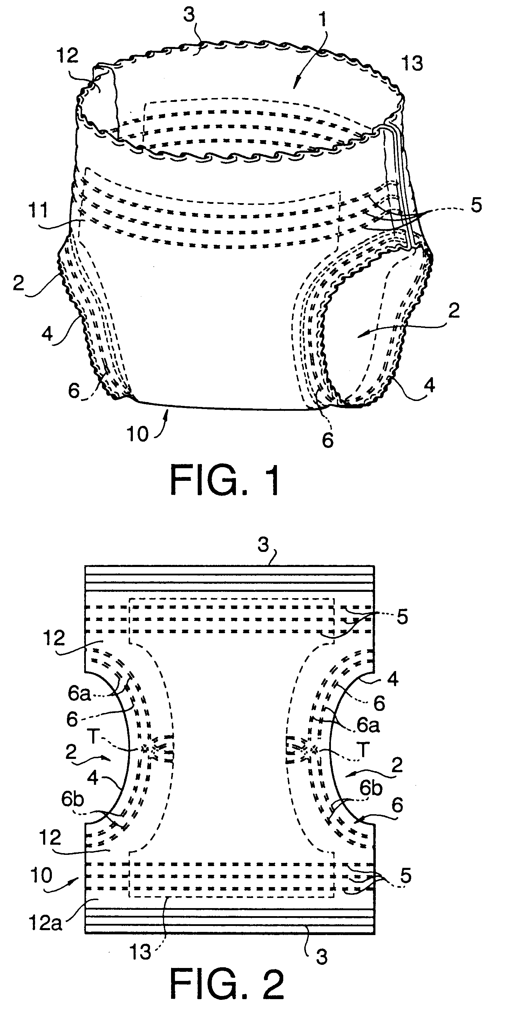

Figure 1 is a perspective view illustrating one embodiment of an absorbent article in accordance with the present invention. Figure 2 is a developed plan view illustrating the absorbent article of Figure 1 prior to bi-folding and sealing the respective side panels thereof to each other.

The absorbent article as illustrated in Figure 1 comprises a main body 10 which has a waist hole 1 and a pair of leg holes 2. The main body 10 includes a waist gather 3 disposed along the periphery of the waist hole 1, and a leg gather 4 disposed along the periphery of each of the leg holes 2. The leg gather 4 is formed by contracting forces of a number of elastic members 6 incorporated therein. A reference numeral 5 illustrates elastic members optionally provided for an improved fit of the main body to a waist portion of a wearer.

The main body 10 comprises a backsheet 11 formed of liquid impermeable material, and a topsheet 12 formed of liquid permeable material, preferably hydrophobic material and an absorbent core 13 interposed therebetween.

In the illustrated embodiment, the elastic members 6 extend inwardly of and along each of right and left, contoured edges of the absorbent article, as shown in Figure 2. One of the important features of the present invention is that the elastic members 6 are positioned between the two sheets of material, such as non-woven fabrics, which form the topsheet. Another important feature is that each set of elastic members is divided or separated at their longitudinal intermediate portions to form two portions 6A, 6B, and respective ends of the two portions 6A, 6B snap back toward the crossing point where they meet to form tails T.

A method for manufacturing the absorbent article as explained above will be now described.

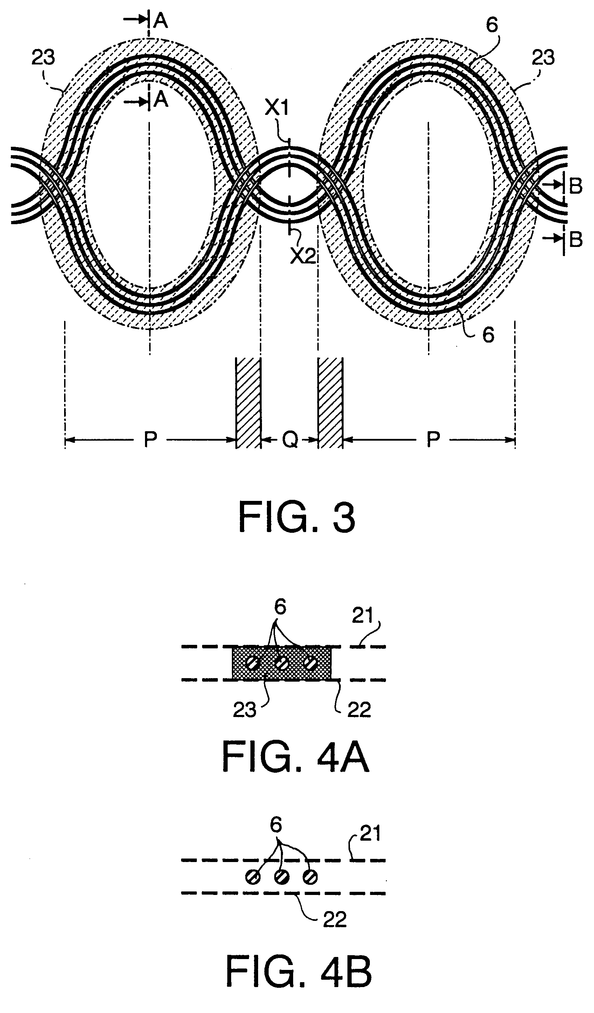

In Figures 3, 4A and 4B, the two sets of elastic members 6 are positioned between two sheets of material, such as two non-woven sheets of fabric 21, 22, and are arranged in overlapping sinusoidal configurations with an inverted phase relation to each other, so that they define a number of oblong areas therebetween. These elastic members 6 are positioned in a uniformly, properly stretched, state and are bonded to the non-woven fabrics in alternate ones of the oblong areas. A reference numeral 23 illustrates bonding, denoted by shading, where the bonding is provided between the tow non-woven fabrics 21, 22, and also between the elastic members 6 and the non-woven fabrics by appropriate bonding means, such as by applying heat and compression, or by the application of adhesives. Reference P indicates bonding regions where the elastic members 6 are bonded to the non-woven fabrics, and reference Q indicates a non-bonding region where the elastic members 6 are not bonded to the non-woven fabrics. The two sets of the elastic members 6 extend along the peripheries of the area that will become leg holes 2 in the bonding regions P, and extend between the adjacent leg holes in the non-bonding regions Q to form cross-over portions.

In the illustrated embodiment, the amplitude of the sinusoidal configuration that each set of the elastic members 6 describes is designed to be greater in the bonding region P than in the non-bonding region Q. This design is intended to decrease length dimensions of the elastic members 6 extending over the non-bonding region Q, so that material cost therefor is reduced, and also length dimensions of the tails T that consequently remain after the division of the elastic members 6 over the region Q are effectively decreased.

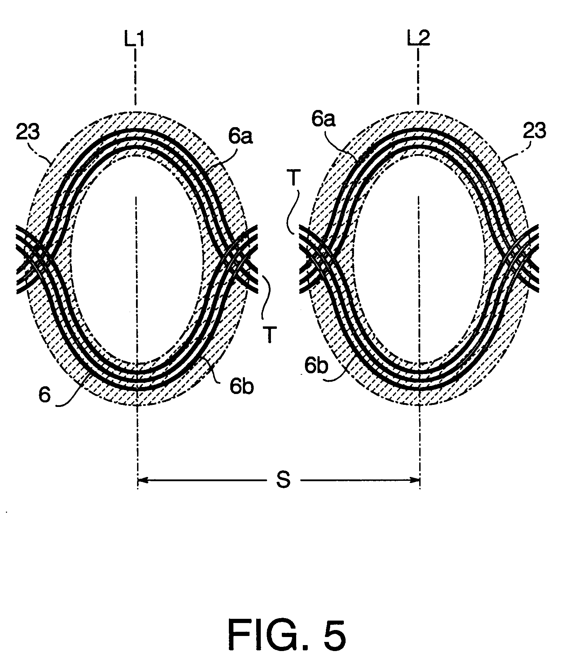

The elastic members 6 as partially secured between the non-woven fabrics 21, 22 are cut out along cutting lines X1, X2 in the cross-over portions in the non-bonding region Q, together with the non-woven fabrics 21, 22. The elastic members 6 are not bonded to the non-woven fabrics 21, 22 over the non-bonding regions Q, so that the cutting allows the elastic members 6 in a stretched state to elastically contract due to elastic recovery thereof, and form short tails T, as illustrated in Figure 5.

When region S is cut out along lines L1, L2, a topsheet is obtained which comprises respective halves of the two adjacent, semi-oblong bonding regions P, and the non-bonding region Q located therebetween.

The cutting of the elastic members simultaneously cuts the non-woven fabrics which enclose the elastic members to form slits therein. Those slits however do not create any adverse results since they are formed in the liquid permeable topsheet.

The elastic members 6 secured to the non-woven fabrics 21, 22 elastically contract due to elastic recovery thereof to form gathers in the region P of the topsheet like conventional articles. The elastic members 6 remain as tails T in the non-bonding region Q (corresponding to the crotch region of the absorbent article) located between the two adjacent bonding regions P. As the tails T are not secured to the non-woven fabrics in the region Q, those non-woven fabrics retain their original flexibility and softness. Accordingly, the topsheet provides an excellent conformity to undulation of a wearer's body.

In the embodiment as illustrated in Figure 3, the amplitude of the sinusoidal configuration that each set of the elastic members 6 describe is selected to be greater in the bonding region P than in the non-bonding region Q. This design permits the quantity and thus the material cost of the elastic members 6 which is not desired in the crotch region to be reduced, and the length dimensions of the tails T that remain after divisions thereof to be decreased.

For the same reason, the stretching rate of the elastic members 6 in the non-bonding regions Q may be increased. For instance, the stretching rate in the non-bonding regions Q is set to about 200% when that in the bonding regions P is selected to be about 100%. Such adjustment of the stretching rate may be made by periodically varying the feed rate of the elastic members.

In another embodiment of the present invention, the cutting of the elastic members 6 simultaneously forms a through aperture in the topsheet.

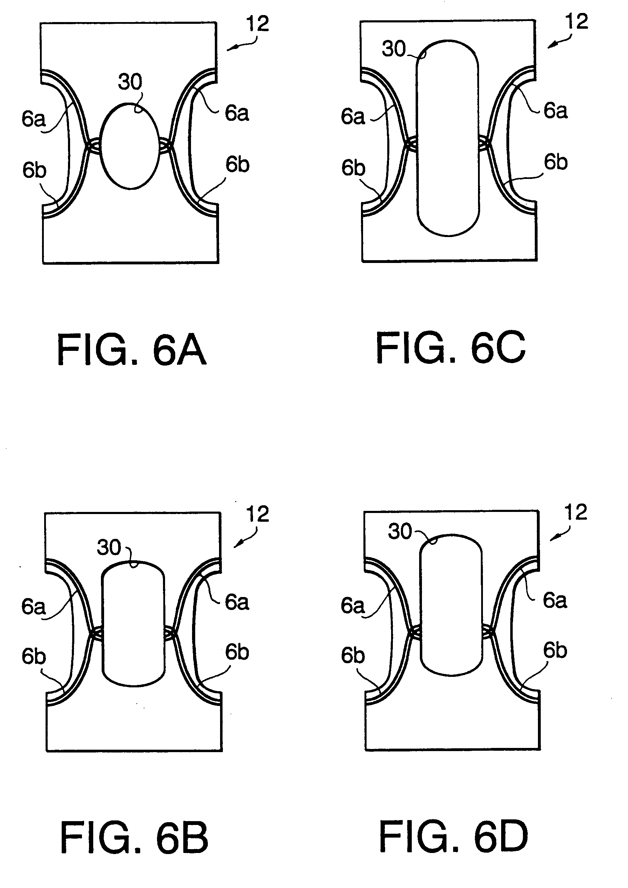

Figures 6A, 6B, 6C, 6D illustrate topsheets 12 respectively having through apertures 30 of various sizes and shapes which cut out both the elastic members 6 and the non-woven fabrics 21, 22.

In Figure 6A, the cutting is applied in a region located between the two adjacent crossing portions where the two portions 6A, 6B of the elastic members meet to form leg gathers 4, so that the two non-woven fabrics 21, 22 and the elastic members 6 are simultaneously cut out to form an oblong or elongated circular through aperture 30. In Figure 6B, the topsheet is illustrated to have the through aperture 30 which is more elongated longitudinally that the aperture 30 of Figure 6A. The through aperture 30 of Figure 6C is more elongated longitudinally than the aperture 30 of Figure 6B. The through aperture 30 of Figure 6D is longitudinally offset toward a front or rear end of the topsheet.

The topsheet with the through aperture 30 is then combined with the backsheet to form an absorbent article. When the absorbent article is applied to a wearer, the through aperture 30 is designed to be placed in facing relation to a crotch region of the wearer. Accordingly, the through aperture 30 serves to allow urine and fecal materials to pass into a space defined between the topsheet 12 and the backsheet 11. This helps provide comfort to the wearer during prolonged use.

Although each of the two sheets of material of the topsheet is described above as comprising a non-woven fabric, the one sheet material positioned in facing relation to the backsheet may alternatively comprise a synthetic resin film, or the like when the through aperture 30 is formed, for example as illustrated in Figures 6A through 6D.

After the topsheet and backsheet are combined material inside the bonded oblong regions of sections P are removed to provide cut-outs to form leg holes in the formed garment. Cuts are made along lines L1, L2 to sever the garment in region S from contiguous garments in the production line. The severed garment then is bi-folded about its transverse center line to the configuration shown in Fig. 1 and side seams are sealed at opposite sides of the waist region to form a garment with the defined waist hole and leg holes.

As explained above, in accordance with the absorbent article of the present invention, the elastic members for forming leg gathers are positioned along the leg holes. Only small parts thereof remain as tails in the crotch region where such elastic members are not required. Accordingly, the absorbent article of the present invention can provide excellent flexibility, a snug fit and comfort to the wearer during use, while effectively preventing leakage from the leg gathers. Furthermore, as the elastic members for forming the leg gathers are not bonded directly to the backsheet, the elastic members are not viewed or observed from outside the absorbent article. This helps provide the article with a better appearance, particularly in its crotch region.

When the through aperture is formed in the crotch region of the topsheet, the article can provide comfort to the wearer as the aperture serves as an inlet of pocket for communicating urine and fecal material to the pocket. In accordance with the present method, such absorbent articles can be readily manufactured in continuous processes, with a reduced material cost of the elastic members.

## Claims

1. An absorbent article comprising:
a main body (10) having a waist hole (1) and a pair of leg holes (2), said main body comprising a topsheet (12) for facing toward a wearer's body, a backsheet (11) disposed outwardly of the topsheet, and an absorbent core (13) interposed between the topsheet and the backsheet;
a waist gather (3) disposed along the waist hole (1);
a leg gather (4) disposed along each of the leg holes (2); two sets of elastic members (6a,6b) each of said two sets of elastic members (6a, 6b) extending along a periphery of a first leg hole (2) from a front end thereof to a midpoint thereof, continuously extending therefrom transversely to a midpoint of a second leg hole (1) to form a cross-over portion, and further extending to a rear end of the second leg hole (2) so that the two sets of elastic members (6a, 6b) are arranged to define an X-shaped configuration,
characterized in that
said topsheet (12) comprises a dual-layered sheet material (21, 22), wherein the two sets of elastic members (6a, 6b) are interposed between the dual sheet material,
said elastic members (6a, 6b) being bonded in a stretched state to said sheet materials along areas that extend along the leg holes so as to exclude the crossover portion from the bonding, said elastic members being cut at the crossover portion so that they snap back toward cross portions of said elastic members to define tail portions (T) extending from the cross portions.

2. The absorbent article of claim 1, wherein
said sheet materials (21, 22) and the elastic members (6a, 6b) are cut out together in the crossover portion, to form an aperture (30) in said topsheet (12).

3. The absorbent article of claim 1 wherein
said topsheet (12) comprises a non-woven fabric disposed to face toward the wearer's body, and a synthetic resin film disposed to face toward the backsheet (11).

4. A method of manufacturing an absorbent article according to claim 1, comprising the steps of:
forming a topsheet (12) by positioning, between two sheets of material (21, 22) in elongated web forms, a pair of elastic members (6a, 6b) in overlapping sinusoidal configurations with an inverted phase relation to each other, so that they define a plurality of elongated oblong areas therebetween,
bonding the elastic members (6a, 6b) to the sheet material (21, 22) in alternate ones of the oblong areas, and
cutting the elastic members (6a, 6b) in the oblong area to which they are not bonded allowing them to snap back toward crossing portions thereof;
combining said topsheet (12) with the backsheet (11); and connecting the absorbent core (13) to the topsheet (12) or backsheet (11) before or after the topsheet (12) and backsheet (11) are combined, and
severing the resultant combination along predetermined cutting lines.

5. The method of claim 4, wherein
said sinusoidal configurations have alternate high and low amplitudes per half cycle, and the elastic members are bonded to the sheet materials in portions having the high amplitude.

## Patentansprüche

1. Absorbierender Artikel, der folgendes aufweist:
einen Hauptkörper (10) mit einem Taillenloch (1) und einem Paar von Beinlöchern (2), wobei der Hauptkörper folgendes aufweist: ein oberes Tuch (12), das einem Körper eines Trägers zugewendet ist, ein Rückseitentuch (11), das vom oberen Tuch aus nach außen angeordnet ist, und ein absorbierendes Kernstück (13), das zwischen dem oberen Tuch und dem Rückseitentuch angeordnet ist;
ein Taillenabschlußbündchen (3), das entlang dem Taillenloch (1) angeordnet ist;
ein Beinabschlußbündchen (4), das jeweils entlang den Beinlöchern (2) angeordnet ist;
zwei Sätze elastischer Elemente (6a, 6b), wobei sich jeder der zwei Sätze elastische Elemente (6a, 6b) entlang eines Umfangs eines ersten Beinlochs (2) von seinem vorderen Ende zu seinem Mittelpunkt erstreckt, sich von da kontinuierlich transversal zu einem Mittelpunkt eines zweiten Beinlochs (1) erstreckt, um einen Kreuzungsteil zu bilden, und sich weiterhin zu einem hinteren Ende des zweiten Beinlochs (2) erstreckt, so daß die zwei Sätze elastischer Elemente (6a, 6b) derart angeordnet sind, daß sie eine X-förmige Konfiguration definieren,
dadurch gekennzeichnet, daß
das obere Tuch (12) ein zweischichtiges Tuchmaterial (21, 22) aufweist, wobei die zwei Sätze elastischer Elemente (6a, 6b) zwischen dem dualen Tuchmaterial angeordnet sind,
wobei die elastischen Elemente (6a, 6b) in einem ausgestreckten Zustand mit den Tuchmaterialien entlang Bereichen verbunden sind, die sich entlang der Beinlöcher derart erstrecken, daß sie den Kreuzungsteil von der Verbindung ausschließen, wobei die elastischen Elemente am Kreuzungsteil derart geschnitten sind, daß sie in Richtung zu Kreuzungsteilen der elastischen Elemente zurückschnappen, um Endteile (T) zu definieren, die sich von den Kreuzungsteilen erstrecken.

2. Absorbierender Artikel nach Anspruch 1, wobei
die Tuchmaterialien (21, 22) und die elastischen Elemente (6a, 6b) miteinander im Kreuzungsteil ausgeschnitten sind, um eine Öffnung (30) im oberen Tuch (12) zu bilden.

3. Absorbierender Artikel nach Anspruch 1, wobei
das obere Tuch (12) eine nicht gewebte Faser aufweist, die derart angeordnet ist, daß sie dem Körper eines Trägers zugewendet ist, und einen synthetischen Kunstharzfilm, der derart angeordnet ist, daß er dem Rückseitentuch (11) zugewendet ist.

4. Verfahren zum Herstellen eines absorbierenden Artikels nach Anspruch 1, das folgende Schritte aufweist:
Ausbilden eines oberen Tuchs (12) durch Positionieren eines Paars elastischer Elemente (6a, 6b) in sich überlagernden sinusförmigen Konfigurationen mit einer invertierten Phasenbeziehung zueinander zwischen zwei Tüchern des Materials (21, 22) in verlängerten Gewebeformen, so daß sie eine Vielzahl verlängerter länglicher Bereiche dazwischen definieren,
Verbinden der elastischen Elemente (6a, 6b) mit dem Tuchmaterial (21, 22) in abwechselnden der länglichen Bereiche, und
Schneiden der elastischen Elemente (6a, 6b) in dem länglichen Bereich, mit dem sie nicht verbunden sind, was ihnen erlaubt, in Richtung zu ihren Kreuzungsteilen zurückzuschnappen;
Kombinieren des oberen Tuchs (12) mit dem Rückseitentuch (11); und
Verbinden des absorbierenden Kernstücks (13) mit dem oberen Tuch (12) oder dem Rückseitentuch (11) vor oder nach der Kombination des oberen Tuchs (12) und des Rückseitentuchs (11), und
Trennen der resultierenden Kombination entlang vorbestimmter Schnittlinien.

5. Verfahren nach Anspruch 4, wobei
die sinusförmigen Konfigurationen abwechselnd hohe und niedrige Amplituden pro Halbzyklus haben, und die elastischen Elemente mit den Tuchmaterialien in Teilen mit der hohen Amplitude verbunden werden.

## Revendications

1. Article absorbant, comprenant :
un corps principal (10) ayant un trou de taille (1) et une paire de trous de jambes (2), ledit corps principal comprenant une feuille supérieure (12) destinée à faire face vers le corps d'un porteur, une feuille postérieure (11) disposée vers l'extérieur de la feuille supérieure, et un noyau absorbant (13) interposé entre la feuille supérieure (12) et la feuille postérieure (11) ;
une fronce de taille (3) disposée le long du trou de taille (1) ;
une fronce de jambe (4) disposée le long de chacun des trous de jambes (2) ;
deux groupes d'éléments élastiques (6a, 6b), chacun desdits deux groupes d'éléments élastiques (6a, 6b) s'étendant le long d'une périphérie d'un premier trou de jambe (2) depuis des extrémités avant de celui-ci jusqu'à un point au milieu de celui-ci, en s'étendant en continu depuis celui-ci transversalement jusqu'à un point au milieu d'un second trou de jambe (1) pour former une partie croisée, et s'étendant en outre vers une extrémité arrière du second trou de jambe (2) de sorte que les deux groupes d'éléments élastiques (6a, 6b) soient agencés pour définir une configuration en forme de X,
caractérisé en ce que
ladite feuille supérieure (12) comprend un matériau en feuille à deux couches (21, 22) dans lesquelles les deux groupes d'éléments élastiques (6a, 6b) sont interposés entre le matériau en feuille double,
et
lesdits éléments élastiques (6a, 6b) sont adhérés dans un état étiré sur lesdits matériaux en feuille le long de zones qui s'étendent le long des trous de jambe de façon à exclure la partie croisée de l'adhésion, lesdits éléments élastiques étant découpés au niveau de la partie croisée de sorte qu'ils retourment élastiquement vers les parties croisées desdits éléments élastiques pour définir des parties de queue (T) qui s'étendent depuis les parties croisées.

2. Article absorbant selon la revendication 1, dans lequel
lesdits matériaux en feuille (21, 22) et les éléments élastiques (6a, 6b) sont découpés ensemble dans la partie croisée, pour former une ouverture (30) dans ladite feuille supérieure (12).

3. Article absorbant selon la revendication 1, dans lequel
ladite feuille supérieure (12) comprend un textile non-tissé disposé de façon à faire face vers le corps d'un porteur, et un film de résine synthétique disposé en face de la feuille postérieure (11).

4. Procédé de fabrication d'un article absorbant selon la revendication 1, comprenant les étapes consistant à :
former une feuille supérieure (12) en positionnant, entre deux feuilles de matériau (21, 22) sous forme de nappes allongées, une paire d'éléments élastiques (6a, 6b) dans des configurations sinusoïdales en chevauchement avec une relation de phase inversée l'un par rapport à l'autre, de sorte qu'ils définissent entre eux une pluralité de zones oblongues allongées,
faire adhérer les éléments élastiques (6a, 6d) au matériau en feuille (21, 22) dans des zones alternées parmi les zones oblongues, et
découper les éléments élastiques (6a, 6b) dans la zone oblongue dans laquelle ils ne sont pas adhérés, en leur permettant de retourner élastiquement vers des parties croisées de ceux-ci ;
combiner ladite feuille supérieure (12) avec la feuille postérieure (11) ;
et connecter le noyau absorbant (13) sur la feuille supérieure (12) ou sur la feuille postérieure (11) avant ou après que la feuille supérieure (12) et la feuille postérieure (11) soient combinées, et
découper la combinaison résultante le long de lignes de découpe prédéterminées.

5. Procédé selon la revendication 4, dans lequel lesdites configurations sinusoïdales ont des amplitudes alternativement fortes et faibles par demi-cycle, et les éléments élastiques sont adhérés sur les matériaux en feuille dans des parties ayant la forte amplitude.
